# EUROPEAN PATENT SPECIFICATION

(11) **EP 1 120 128 B1**
(45) Date of publication and mention of the grant of the patent: **09.11.2005**
(21) Application number: 00610014.3
(22) Date of filing: 28.01.2000
(51) Int. Cl.: A61M 25/00

(54) **A catheter system**
Kathetersystem
System de catheter

(43) Date of publication of application: 01.08.2001
(73) Proprietor: WILLIAM COOK EUROPE ApS, 4632 Bjaeverskov (DK); Cook Incorporated, Bloomington, IN 47402-0489 (US)
(72) Inventor: Klint, Henrik Sonderskov, 2800 Lyngby (DK)
(74) Representative: Indahl, Peter Jensen

(56) References cited:
- WO-A-93/05842
- WO-A-98/58696
- US-A- 5 695 483

## Description

The present invention relates to a catheter system comprising a catheter having a distal end, a body portion and at least one lumen extending through the body portion in the longitudinal direction from a proximal end towards the distal end, and at least one central member for co-axial advancement through the catheter lumen, which body portion is made of a multifilar helically wound row of wires.

Catheters for medical diagnostic or therapeutic use are well known. A wide variety of catheters exists for percutaneous insertion by the Seldinger technique into the vascular system for placing agents or devices at specific locations in the vascular system. The vessels of the peripheral vasculature have a relatively large diameter and low tortuosity, the coronary vasculature is somewhat smaller and more tortuous, and the vasculature in the soft tissue of the brain and liver is of small lumen and is very tortuous.

In order to be able to access the various parts of the vasculature the catheter needs to be flexible and to maintain its column strength when it follows a tortuous path. When the catheter has been correctly positioned the central member is to be pushed through the catheter lumen. The more tortuous the path and the smaller the catheter the more difficult it is to advance the central member through the catheter lumen. This difficulty is in particular pronounced in co-axial systems for intercranial use where interventional neurological techniques include delivery of a central member for use as an vaso-occlusive agent, treatment of tumors, aneurysms, AVS (areriovenous-shunts) etc.

In the present context the term catheter is to be understood in the sense that it can be an ordinary catheter, but also a sheath, which is a short catheter, and in the latter case the central member can be a catheter, e.g. a catheter according to the present invention. The sheath can have a check valve or a fitting at the proximal end in order to stop bleeding out of the puncture site.

WO 98/58696 discloses a catheter of the above-mentioned kind and for use as a epidural catheter. In this catheter the wires act as a reinforcement to prevent tensile failure of the catheter.

It is an object of the present invention to provide a catheter system which makes it easier to advance the central member through the catheter also in cases where the catheter exhibits sharp turns.

In view of this, the catheter according to the invention is characterized in that the inside surface of said body portion is mainly undeformable by the central member, and that the wires in said row are machined to a lesser outer diameter, e.g. by grinding, in a region of the catheter.

By making the body portion of a row of two or more wires, which row is helically wound with a pitch roughly corresponding to the width of the adjacent wires in the row, the wound wires transfer torque and also force components directed in the axial direction of the catheter to the distal end thereof, and this construction is found to give a very high resistance to kinking of the catheter. When the catheter is heavily bent the cross-section of the catheter maintains a circular shape. This is an distinct advantage over prior art catheters which are deformed into oval shape when bent, and thus they are much more prone to kinking. The catheter surprisingly maintains its capabilities for transferring torque and push when it follows a tortuous path involving two or more loops, probably because of the good kinking resistance. These qualities facilitate placement of the catheter at the desired position in the vascular system, and by making the catheter system so that the inner surface of the catheter is mainly undeformable by the central member it is virtually impossible for the central member to get stuck in the catheter wall, even in situations where the catheter is heavily curved. This is in contrast to prior art co-axial systems where the catheter is made of a soft material such as a resin, the inner surface of which is readily deformable in a local area, causing the formation of a small bead in front of the tip of the central member bearing against the wall of the curved catheter. It is an advantage of the catheter according to the present invention that the wall is primarily made of wires that provide a hard and relatively slippery inner surface resulting in low resistance to advancing the central member through the lumen of the catheter.

If the catheter is placed in the vasculature without using a guidewire in a soft tissue region it is preferred that the distal end of the catheter is provided with a buffer member, such as a soft obturator. The buffer member distributes the force from the catheter tip over a large area so that damage to the vascular wall is avoided.

In one embodiment the row of wires is made up of from 2 to 12 helically wound wires, preferably of from 4 to 8 helically wound wires. By using several wires their aggregate width can be adapted to correspond to the desired pitch for the given diameter of the catheter. A row of more than 12 wires has a tendency to buckle when the wires are helically wound in the common winding operation. For wires of round cross-sectional shape a number of from 4 to 8 wires in the row is preferred, but for flat wires or wires of oval shape two or three wires in a row can be more suitable.

In order to promote uniform and well-defined characteristics of the catheter along its length, the wires in the row can be located closely next to each other so that they mutually support each other. In this manner a possible deflection of a single wire strand is reduced to a minimum by the other wires in the row. As the wires in the row are wound into a helical course in a common movement there can be an interstice between the turns of the row of wires. The inside surface of the catheter is also more even which promotes advancing of the central member.

In an embodiment the wires in said row have a pitch angle in the range of 26°-76°, preferably a pitch angle in the range of 40°-65°. Although it is possible to use other pitch angles, angles chosen in these ranges provide a balanced solution to the requirements for the desired high flexibility, high column strength and fine torqueability. The inner range of 40°-65° is in particular useful for manipulation of the catheter into very distant, small sized vessels, such as in blood vessels in the brain, whereas the subrange of 35°-40° is applicable when very high flexibility is a requirement, and the subrange of 70°-76° is applicable when very high pushability is a requirement. It is of course possible to choose different pitch angles in different segments of the catheter.

At the time of performing the winding operation of the body portion, the individual wires in the row wound in the helical pattern have preferably a mainly circular cross-section. This facilitates the winding operation because twisting of a wire does not result in disorder in the row.

The central member is preferably associated with a pusher member extending out of the proximal end of the catheter and arranged in the catheter lumen for co-axial advancement therethrough.

It is possible to provide the catheter with a coating on the inner surface of the body portion which will provide a more even inner surface against which the central member slides and result in a catheter of a very small wall thickness relative to its diameter.

In an embodiment the coating is a low-friction coating, such as a PTFE coating. A low-friction coating applied on the external side of the catheter wall acts to reduce the forces required to push forward the catheter inside a larger guiding catheter or a sheath, and a low-friction coating applied on the internal side of the catheter wall acts to reduce the forces required to push forward a guidewire or central member advanced through the catheter.

In yet an embodiment the coating is a hydrophilic coating. The lubricating effect of the coating can be used to enhance the effects provided by the main features of the invention, but often such a coating is not necessary because the central member slides easily along the catheter wall.

When a coating is used, it is preferably thin and constitutes only a minor part of the wall thickness of the body portion. The thickness of the coating at the middle of the wire can be less than 0.1 mm, and preferably it is less than 0.02 mm.

It is possible to promote the flexibility of the catheter by machining the wires in said row to a lesser outer diameter, e.g. by grinding, at a region of the catheter. The region can extend along the whole length of the body portion, so that it is given a very precise outer dimension by the machining. In another embodiment the region is a distal region machined to a tapering shape with decreasing outer diameter in the distal direction causing the catheter to have an increasing flexibility towards the distal end which promotes introduction into very diminutive vessels. The reduced cross-sectional area of the wires produced by the machining greatly increases the bending flexibility of the catheter system without sacrificing its ability to transfer torque.

It is preferred that the catheter system at least in a 30 cm long distal area has a maximum outer diameter of less than 2.0 mm. A maximum diameter of 2 mm in the part of the catheter advanced through the vascular system allows for a straightforward percutaneous introduction by the Seldinger technique and easy navigation through the curves in the larger vessels. A maximum diameter of less than 1.00 mm allows introduction into quite fine and diminutive vessels such as into the external and internal carotid arteries. It is further possible to restrict the maximum outer diameter to at the most 0.75 mm which makes it possible to easily advance the catheter into e.g. the liver or other soft tissue areas, and by keeping the maximum outer diameter below 0.30 mm in a distal end area having a length of at least 10 cm even the most distant vascular regions are accessible and the catheter is excellent as a neuro-microcatheter.

In a further embodiment the number of wires in said helically wound row of wires varies along the length of the catheter. This can be attained by reducing, during the winding operation, the number of wires in the row. The lower number of wires in the row can be utilized to wind the row of wires with a larger pitch angle which increases the flexibility of the catheter. It is preferred that the number of wires diminishes in the distal direction so that the softness of the catheter increases without any change of material and without bonding together several separate catheter segments.

When the catheter system has to traverse large lumen vascular paths in order to reach the more difficult small size vascular vessels, the helically wound row of wires can be stiffened in a proximal segment of said body portion by a supplementary tubular member, such as a cannula.

In the following, examples of the invention are descriped in more detail with reference to the very schematic drawings, on which
Fig. 1 is a side view of a catheter system according to the present invention,
Figs. 2 and 3 are enlarged partial views in longitudinal section of embodiments of the catheter system in Fig. 1,
Fig. 4 is a partial view in longitudinal section of an embodiment where the number of wires in a row varies along the length of the catheter,
Fig. 5 is an enlarged partial and sectional view of the transition between two catheter segments having wires of different diameter,
Fig. 6 is an enlarged view of an embodiment having a catheter tip with a buffer member,
Fig. 7 depicts a winding operation on a multifilar row of wires,
Fig. 8 depicts a catheter segment having decreasing outer diameter,
Fig. 9 is an illustration of the catheter system in position in the vascular system, and

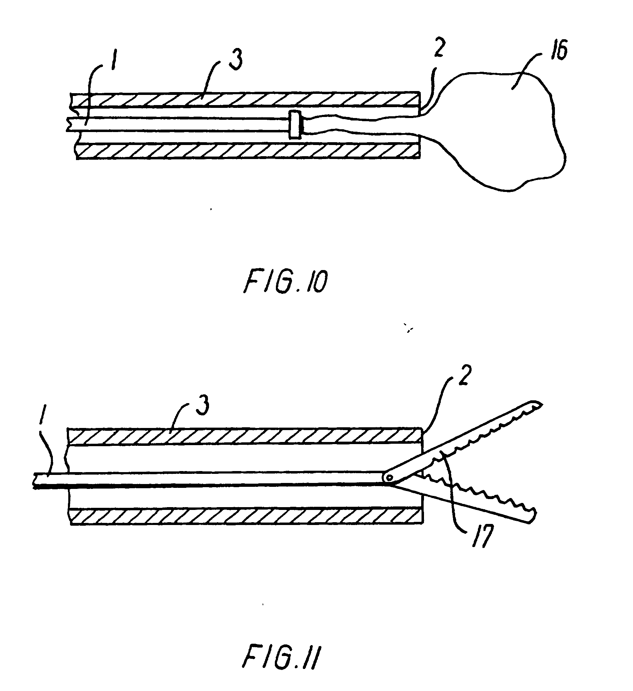

Fig. 10 and 11 are enlarged views of central members being advanced our of the distal end of the catheter.

In the following description of the depicted embodiments the same reference numerals are used for features of the same type.

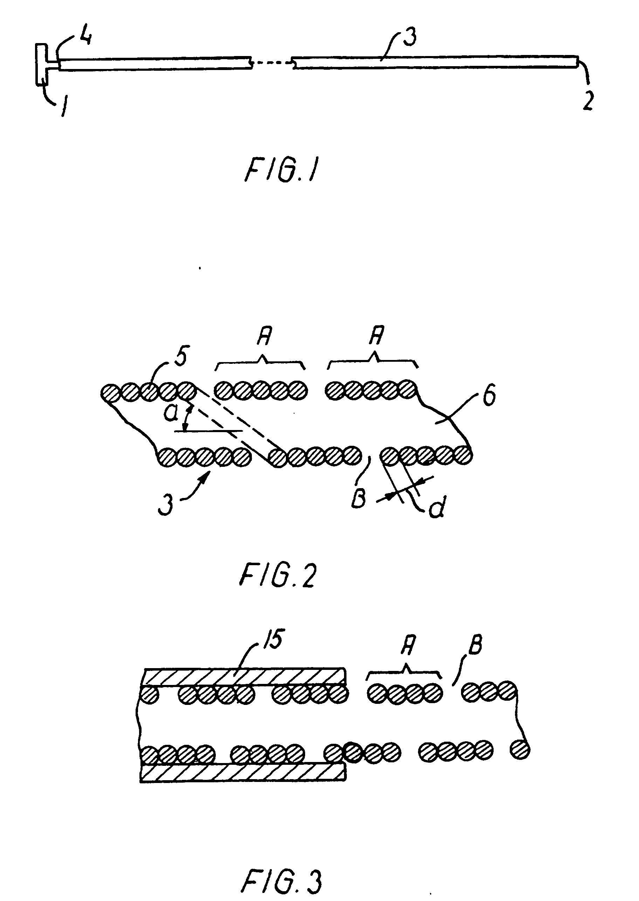

A catheter system illustrated in Fig. 1 includes a pusher 1 for a central member, and a catheter having a distal end 2 and a body portion 3 extending from the distal end to a proximal end 4. The body portion portion is made of a first helically wound multifilar row of wires 5 and it has a central longitudinally extending lumen 6. The catheter is normally open ended at both the proximal and the distal end, but for special uses, such as a single lumen balloon dilation catheter the distal end can be provided with means for barring the distal end opening.

For example, the catheter system according to the present invention can be a single lumen balloon dilation catheter for percutaneous transluminal coronary angioplasty, where the central member is used to block the distal opening during inflation of the balloon. The catheter system can also be for placing the central member in the vascular system. To give some examples, the central member can be an embolization means in form of a sack containing several occlusion coils, it can be a stent for expansion on a balloon, it can be a sensor body for measuring pressure or temperature or the composition of blood, it can be a physical shunt member, it can be a retrieval wire or a forceps used to retrieve another member from a vascular site, or it can be a central member of some other kind.

The wires 5 used in the helically wound multifilar row are of a linear elastic material, such as stainless steel, titanium or tantalum, or it is made of a superelastic alloy, such as Nitinol. Preferably, the wires have an ultimate tensile strength in the range of 1800-2700 N/mm² but lower or higher values are also possible. The body portion 3 of the catheter is made by placing a group of from two to twelve wires in a row next to each other, e.g. according to the desired pitch angle, whereafter the group of wires is wound. Because a row of wires is wound, the individual wire is restricted in movement by the other wires and is plastically deformed into a permanent helical shape which is kept without any further restraints than the remaining wires in the row. The winding can be done on the inside end of a tubular support member where the row of wires are inserted at said end by rotating and simultaneously pushing the wires against the inside of the support. The wound wire then exits at the other end of the support. This produces a wire body with a very precise outer diameter.

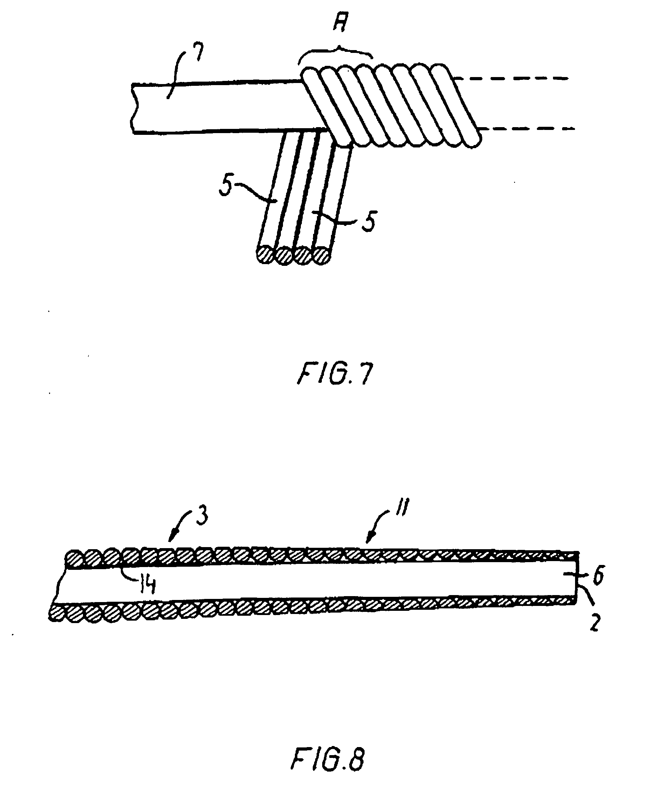

Alternatively, the winding operation can take place about a mandrel 7. about a mandrel 7. Fig. 7 depicts winding of a row A of five identical wires 5. After the winding the mandrel with the coiled wires can be subjected to heat treatment in order to remove residual stresses from the wires. As an example the heat treatment can last for about two hours in an oven at a temperature of about 500°C. Generally, the temperature can be in the range of 400-600°C and the holding time at the temperature can last for many hours, such as up till 20 hours or more. After the heat treatment the mandrel is removed from the wires. The resulting helically wound multifilar row of wires maintain their mutual position even when heavily torqued, bent or pushed, presumably because each single wire is supported by the contiguous wires in the row. The winding operation can be effected so that the windings are touching each other, but preferably it is performed so that an interstice B is present between the turns (Fig. 2). The interstice facilitates bending of the body portion in thight turns.

The size of the pitch angle a depends on the diameter of the wires, the diameter of the body portion 3 and the number of wires in the row. The most preferred pitch angle a for the catheter is in the range of 40° - 65° or 50° - 70°. However, the combination of torque-transferral, pushability and transverse flexibility is normally well-balanced for pitch angles in the range of 50-68°. The diameter d of the wire is typically in the range of 0.06-0.75 mm, and preferably in the range of 0.15-0.45 mm.

In order to make the tip portion of the catheter more visible on a screen, it is desirable to use some kind of radiopaque material, such as platinum or gold. It can be of annular shape and be located at a predetermined distance from the distal end 2, or the terminal end of the distal tip of the catheter can be provided with a marker means for making it radiopaque, e.g. a gold layer or a gold thread.

The catheter can be made with uniform diameter throughout its length. In case the catheter has diminishing diameter towards the distal end a prefabricated catheter of uniform diameter can be ground to the desired dimensions.

As an alternative or supplement to grinding the catheter can be composed of several segments in which the wires have mutually different diameters and cross-sectional areas. In a proximal segment 8 the wires can have a larger diameter than the wires in a distal segment 9. The segments can be joined together in axial extension by laser welding 10 as depicted in Fig. 5, by soldering, by bracing or in another manner such as mutual geometrically locking of the wires in the segments or by mechanical locking, such as press-fitting one segment into the lumen of the other segment, or binding the segments in axial extension with threads or suture.

When the catheter body is of multi-segment construction the inner lumens of the segments are preferably of even size which brings the advantage that an advancing guidewire can not grip onto a step in the inner wall of the body portion.

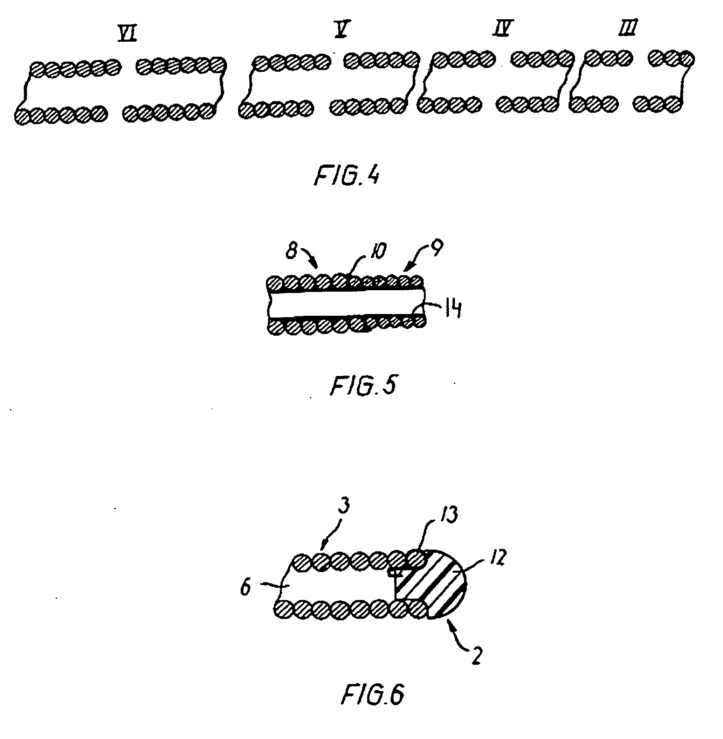

In the embodiment illustrated in Fig. 4 the number of wires in said helically wound row of wires varies along the length of the catheter. During the winding operation the number of wires in the row is reduced one by one at the points in time where the individual segment having an even number of wires has obtained the desired length. The segment marked VI has six wires in the row, and the segments marked V, IV and III have five, four and three wires, respectively, in the row. Each time a wire is left out of the row, the pitch gets shorter and the pitch angle grows resulting in a even more flexible consecutive segment. The advantage of this embodiment is that the wires extending into the distal end segment are continuous from the distal end to the proximal end of the catheter, thus avoiding any need for joining the various segments. It is possible to secure the thread ends of the discontinous wires onto the other wires, such as by welding, soldering, etc.

A grinding procedure can also be used to produce a tapered segment 11 in the body portion portion 3. The taper can extend along a substantial length of the body portion. In the tapered segment the outer diameter of the catheter diminishes towards the distal end. Due to the taper the catheter obtains a gradually increasing transverse flexibility and a higher softness, but column stength and torque are nevertheless surprisingly transferred to the distal end.

When the catheter is to be advanced without a guidewire the distal end 2 can be provided with a soft buffer 12 having a rounded distal end which acts gently on the vascular wall when the catheter is pushed forward. A thread 14 can be securely embedded into the soft pliable material of buffer 12 and be ensnared around one of the distal wires, so that the thread will keep the buffer connected to the catheter body portion when the buffer is pushed out and cleared from the lumen of the catheter.

The wound wires 5 can be provided with a coating 14 on the inside or on the outside or on both sides of the catheter body. The coating is relatively thin and is preferably made of an elastic material which can be hydrophilic. The coating extends along the entire length of the catheter and is typically applied after winding and heat treatment of the catheter body have been completed. As an example the coating can be of PTFE applied onto the outside of the body portion in the same manner as such a coating is traditionally applied onto the exterior of a guidewire. When the coating is to be applied on the external and the internal sides of the body portion the catheter length can be dipped into a bath of liquid coating material which is then allowed to solidify.

In case it is desirable to use a hydrophilic coating, the coating can e.g. comprise a hydrophilic polymer selected from the group comprising polyacrylate, copolymers comprising acrylic acid, polymethacrylate, polyacrylamide, poly(vinyl alcohol), poly(ethylene oxide), poly(ethylene imine), carboxymethylcellulose, methylcellulose, poly(acrylamide sulphonic acid), polyacrylonitril, poly(vinyl pyrrolidone), agar, dextran, dextrin, carrageenan, xanthan, and guar. The hydrophilic polymers can comprise ionizable groups such as acid groups, e.g. carboxylic, sulphonic or nitric groups. The hydrophilic polymers may be cross-linked through a suitable cross-binding compound. A cross-binder generally comprise two or more functional groups which provides for the connection of the hydrophilic polymer chains. The actual used cross-binder depends on the polymer system. If the polymer system is polymerized as a free radical polymerisation, a preferred cross-binder comprises 2 or 3 unsaturated double bonds.

In the follwing some examples of catheter systems made according to the invention are described.

### EXAMPLE 1:

A catheter was made of a helically wound row of 4 wires of 0.35 mm wire diameter d. The body portion of wound wires had initially an outside diameter of 1.67 mm and an inner lumen of 0.97 mm. A coating of PTFE of a minimum thickness of 0.1 mm was applied onto the inside of the catheter. The catheter was set in a complex curved shape involving three consecutive loops of a loop diameter of 24 mm axially separated by two loops of a loop diameter of 18 mm and a number of further turns representative of a complex vascular structure. Then a bag 16 with four occlusion coils was pushed forward by the pusher 1 until it discharged through the distal opening 2. There was no noticeable sticking of the bag 16 against the inside of the catheter.

### EXAMPLE 2:

A catheter was made of a helically wound row of 5 wires of 0.30 mm wire diameter d. The winding of a first segment of the body portion was made with an outside diameter of 1.20 mm and an inner lumen of 0.6 mm. Another segment was made up of a helically wound row of four wires of 0.15 mm wire diameter. This segment had a length of 20 cm and an outside diameter of 1.20 mm and an inside diameter of 0.9 mm. The segments were jointed by laser welding. The catheter body was provided with a PTFE coating on its outside. The catheter was advanced through a complex curved vascular system involving several consecutive, retrograde turns in vessels having a lumen of only 2 mm and less. Then a pair of forceps 17 was advanced through the catheter, activated to grab the desired item, such as a kidney stone, and retracted through the catheter lumen.

### EXAMPLE 3:

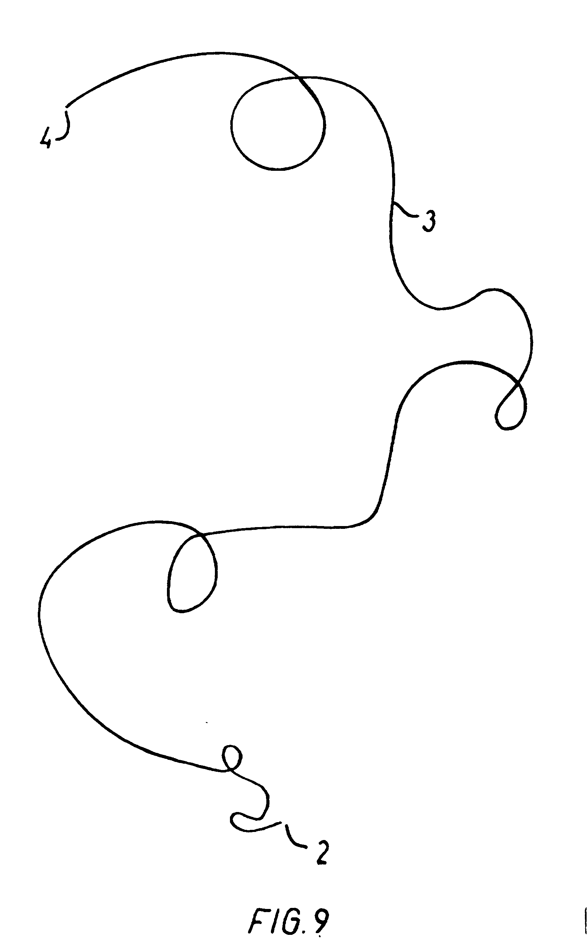

A catheter was made of a first helically wound row of 8 wires of 0.075 mm wire diameter d. The winding was made with an outside diameter of 0.25 mm and an inner lumen of 0.1 mm. The body portion had a length of 160 cm and was uncoated. When tested the catheter showed no problems. After placing the catheter in a very complex pattern involving several sharp turns (see an example in Fig. 9) a guidewire could be advanced with only very low friction, and after removal of the guidewire, central members in the form of fluid injected embolization coils were delivered through the catheter.

When the catheter system is to be introduced into the vascular system there is firstly established a percutaneous puncture site, e.g. by the Seldinger technique or an existing puncture site is used. Then the body portion of the catheter is inserted through the cannula, sheat or hemostatic valve at the puncture site and the catheter is advanced and navigated through the vascular system to the actual site. Due to the very high flexibility, pushability and torqueability of the catheter it can be advanced to the site without use of a guidewire, or a sheat to negotiate the sharp curves in the path.

When large lumen vessels are to be traversed in order to enter the vasculature near the target site, it can be an advantage to stiffen the proximal portion of the catheter by inserting it through a cannula 14 ( Fig. 3) or another kind of a more rigid structure.

Individual features of the various embodiments can be combined into further embodiments according to the present invention. It is possible to effect the sealing coating as a multilayer coating, e. g. comprising a primer-coating and a top-coat where the primer-coating is chosen to provide a strong bonding to the wires, and the top-coat can be a hydrophilic slippery coating providing a low friction surface.

## Claims

1. A catheter system comprising a catheter having a distal end (2), a body portion (3) and at least one lumen (6) extending through the body portion in the longitudinal direction from a proximal end (4) towards the distal end (2), and at least one central member for co-axial advancement through the catheter lumen (6), which body portion (3) is made of a multifilar helically wound row (A) of wires (5), **characterized in that** the inside surface of said body portion (3) is mainly undeformable by the central member, and that the wires (5) in said row (A) are machined to a lesser outer diameter, e.g. by grinding, in a region of the catheter.

2. A catheter system according to claim 1, **characterized in that** the row (A) of wires is made up of from 2 to 12 helically wound wires (5), preferably of from 4 to 8 helically wound wires (5).

3. A catheter system according to claim 2, **characterized in that** the wires (5) in the row (A) are located closely next to each other.

4. A catheter system according to any one of claims 1-3, **characterized in that** the wires (5) have a pitch angle (α) in the range of 26°-76°, preferably a pitch angle (α) in the range of 40°-65°.

5. A catheter system according to any one of claims 1-4, **characterized in that** the body portion (3) is wound of wires (5) having a mainly circular cross-section.

6. A catheter system according to any one of claims 1-5, **characterized in that** the central member is associated with a pusher member (1) arranged in the lumen (6) for co-axial advancement through the catheter.

7. A catheter system according to any one of claims 1-6, **characterized in that** the catheter is provided with a coating (14) on the inner surface of the body portion (3).

8. A catheter system according to claims 7, **characterized in that** the coating (14) is a low-friction coating, such as a PTFE coating.

9. A catheter system according to any one of claims 1-8, **characterized in that** the coating (14) is a hydrophilic coating.

10. A catheter according to claim 9, **characterized in that** the thickness of the coating (14) at the middle of the wire (5) is less than 0.1 mm, preferably less than 0.02 mm.

11. A catheter according to any one of the claims 1-10, **characterized in that** said region is a distal segment (11) machined to a tapering shape with decreasing outer diameter in the distal direction.

12. A catheter according to any one of the preceding claims, **characterized in that** the catheter at least in a 30 cm long distal segment (11) has a maximum outer diameter of less than 2.0 mm.

13. A catheter according to any one of the claims 1-12, **characterized in that** the catheter is a micro-catheter with a 30 cm long distal segment (11) having a maximum outer diameter of less than 1.00 mm, preferably at the most 0.75 mm.

14. A catheter according to any one of the claims 1-13, **characterized in that** the catheter is a neuro-microcatheter having a distal segment (11) of a length of at least 10 cm which has a maximum outer diameter of less that 0.30 mm.

15. A catheter according to any one of the claims 1-14, **characterized in that** the number of wires (5) varies along the length of the catheter, preferably so that the number of wires (5) diminishes in the distal direction.

16. A catheter according to claim 14 or 15, **characterized in that** in a proximal segment the row of wires (5) is stiffened by a supplementary tubular member (15).

17. A catheter according to any one of the preceeding claims , **characterized in that** the wires (5) in the helically wound row (A) are located closely next to each other, and that the row (A) of wires has been wound into the helical course in a common movement, possibly with an interstice (B) between the turns of the row of wires.

18. A catheter according to any one of the preceeding claims , **characterized in that** the body portion is made of a single helical wound row (A) of wires.

19. A catheter according to any one of the preceeding claims, **characterized in that** wires (5) extend continuously from the distal end (2) to the proximal end (4) of the catheter.

## Patentansprüche

1. Kathetersystem, das umfasst: einen Katheter mit einen distalen Ende (2), einem Körperabschnitt (3) und wenigstens einem Lumen (6), das sich durch den Körperabschnitt in Längsrichtung von einem proximalen Ende (4) zu dem distalen Ende (2) erstreckt, und wenigstens ein mittleres Element, das sich koaxial durch das Katheter-Lumen (6) fortbewegen soll, wobei der Körperabschnitt (3) aus einer mehrfädigen, schraubenlinienförmig gewickelten Lage (A) von Drähten (5) hergestellt ist, **dadurch gekennzeichnet, dass** die Innenoberfläche des Körperabschnitts (3) durch das mittlere Element im Wesentlichen nicht verformbar ist und dass die Drähte (5) in der Lage (A) in einem Bereich des Katheters, etwa durch Schleifen, auf einen kleineren äußeren Durchmesser bearbeitet sind.

2. Kathetersystem nach Anspruch 1, **dadurch gekennzeichnet, dass** die Lage (A) von Drähten aus 2 bis 12 schraubenlinienförmig gewickelten Drähten (5), vorzugsweise aus 4 bis 8 schraubenlinienförmig gewickelten Drähten (5) hergestellt ist.

3. Kathetersystem nach Anspruch 2, **dadurch gekennzeichnet, dass** die Drähte (5) in der Lage (A) eng nebeneinander liegen.

4. Kathetersystem nach einem der Ansprüche 1-3, **dadurch gekennzeichnet, dass** die Drähte (5) einen Steigungswinkel (α) im Bereich von 26°-76°, vorzugsweise einen Steigungswinkel (α) im Bereich von 40°-65° besitzen.

5. Kathetersystem nach einem der Ansprüche 1-4, **dadurch gekennzeichnet, dass** der Körperabschnitt (3) aus Drähten (5) mit einem im Wesentlichen kreisförmigen Querschnitt gewickelt ist.

6. Kathetersystem nach einem der Ansprüche 1-5, **dadurch gekennzeichnet, dass** dem mittleren Element ein in dem Lumen (6) angeordnetes Schiebeelement (1) zugeordnet ist, das sich durch den Katheter koaxial fortbewegen soll.

7. Kathetersystem nach einem der Ansprüche 1-6, **dadurch gekennzeichnet, dass** der Katheter auf der inneren Oberfläche des Körperabschnitts (3) mit einer Beschichtung (14) versehen ist.

8. Kathetersystem nach Anspruch 7, **dadurch gekennzeichnet, dass** die Beschichtung (14) eine Beschichtung mit niedriger Reibung, etwa eine PTFE-Beschichtung, ist.

9. Kathetersystem nach einem der Ansprüche 1-8, **dadurch gekennzeichnet, dass** die Beschichtung (14) eine hydrophile Beschichtung ist.

10. Katheter nach Anspruch 9, **dadurch gekennzeichnet, dass** die Dicke der Beschichtung (14) in der Mitte des Drahts (5) kleiner als 0,1 mm, vorzugsweise kleiner als 0,02 mm ist.

11. Katheter nach einem der Ansprüche 1-10, **dadurch gekennzeichnet, dass** der Bereich ein distales Segment (11) ist, das in eine sich verjüngende Form mit in distaler Richtung abnehmendem Außendurchmesser bearbeitet ist.

12. Katheter nach einem der vorhergehenden Ansprüche, **dadurch gekennzeichnet, dass** der Katheter wenigstens in einem 30 cm langen distalen Segment (11) einen maximalen Außendurchmesser von weniger als 2,0 mm hat.

13. Katheter nach einem der Ansprüche 1-12, **dadurch gekennzeichnet, dass** der Katheter ein Mikrokatheter mit einem 30 cm langen distalen Segment (11) ist, das einen maximalen Außendurchmesser von weniger als 1,00 mm, vorzugsweise höchstens 0,75 mm, besitzt.

14. Katheter nach einem der Ansprüche 1-13, **dadurch gekennzeichnet, dass** der Katheter ein Neuro-Mikrokatheter mit einem distalen Segment (11) mit einer Länge von wenigstens 10 cm ist, das einen maximalen Außendurchmesser von weniger als 0,30 mm hat.

15. Katheter nach einem der Ansprüche 1-14, **dadurch gekennzeichnet, dass** die Anzahl der Drähte (5) über die Länge des Katheters unterschiedlich ist, vorzugsweise in der Weise, dass die Anzahl der Drähte (5) in distaler Richtung abnimmt.

16. Katheter nach Anspruch 14 oder 15, **dadurch gekennzeichnet, dass** die Lage aus Drähten (5) in einem proximalen Segment durch ein zusätzliches röhrenförmiges Element (15) versteift ist.

17. Katheter nach einem der vorhergehenden Ansprüche, **dadurch gekennzeichnet, dass** die Drähte (5) in der schraubenlinienförmig gewickelten Lage (A) eng nebeneinander angeordnet sind und dass die Lage (A) aus Drähten in einer gemeinsamen Bewegung in die schraubenlinienförmige Bahn gewickelt sind, möglicherweise mit einem Zwischenraum (B) zwischen den Windungen der Lage aus Drähten.

18. Katheter nach einem der vorhergehenden Ansprüche, **dadurch gekennzeichnet, dass** der Körperabschnitt aus einer einzigen schraubenlinienförmig gewickelten Lage (A) aus Drähten hergestellt ist.

19. Katheter nach einem der vorhergehenden Ansprüche, **dadurch gekennzeichnet, dass** sich die Drähte (5) ununterbrochen von dem distalen Ende (2) zu dem proximalen Ende (4) des Katheters erstrecken.

## Revendications

1. Système de cathéter comprenant un cathéter comportant une extrémité distale (2), une partie formant le corps (3) et au moins une lumière (6) s'étendant à travers la partie formant le corps dans le sens longitudinal à partir d'une extrémité proximale (4) vers l'extrémité distale (2), et au moins un membre central pour une progression coaxiale à travers la lumière (6) de cathéter, laquelle partie formant le corps (3) est constituée d'une rangée (A) à enroulement hélicoïdal multifilaire de fils (5), **caractérisé en ce que** la surface interne de ladite partie formant le corps (3) est essentiellement indéformable par le membre central, et que les fils (5) dans ladite rangée (A) sont façonnés à un diamètre externe plus petit, par exemple par meulage, dans une région du cathéter.

2. Système de cathéter selon la revendication 1, **caractérisé en ce que** la rangée (A) de fils est composée de 2 à 12 fils enroulés hélicoïdalement (5), de préférence de 4 à 8 fils enroulés hélicoïdalement (5).

3. Système de cathéter selon la revendication 2, **caractérisé en ce que** les fils (5) dans la rangée (A) sont situés à proximité les uns à côté des autres.

4. Système de cathéter selon l'une quelconque des revendications 1 à 3, **caractérisé en ce que** les fils (5) ont un angle de pas (α) dans la plage de 26 ° à 76 °, de préférence un angle de pas (α) dans la plage de 40 ° à 65 °.

5. Système de cathéter selon l'une quelconque des revendications 1 à 4, **caractérisé en ce que** la partie formant le corps (3) est un enroulement de fils (5) ayant une section essentiellement circulaire.

6. Système de cathéter selon l'une quelconque des revendications 1 à 5, **caractérisé en ce que** le membre central est associé avec un membre poussoir (1) arrangé dans la lumière (6) pour progression coaxiale à travers le cathéter.

7. Système de cathéter selon l'une quelconque des revendications 1 à 6, **caractérisé en ce que** le cathéter est pourvu d'un revêtement (14) sur la surface interne de la partie formant le corps (3).

8. Système de cathéter selon la revendication 7, **caractérisé en ce que** le revêtement (14) est un revêtement à faible frottement, tel qu'un revêtement en PTFE.

9. Système de cathéter selon l'une quelconque des revendications 1 à 8, **caractérisé en ce que** le revêtement est un revêtement hydrophile.

10. Cathéter selon la revendication 9, **caractérisé en ce que** l'épaisseur du revêtement (14) au milieu du fil (5) est inférieure à 0,1 mm, de préférence inférieure à 0,02 mm.

11. Cathéter selon l'une quelconque des revendications 1 à 10, **caractérisé en ce que** ladite région est un segment distal (11) façonné en une forme conique avec un diamètre externe décroissant dans la direction distale.

12. Cathéter selon l'une quelconque des revendications précédentes, **caractérisé en ce que** le cathéter, au moins, dans un segment distal (11) d'une longueur de 30 cm, a un diamètre externe maximal inférieur à 2,0 mm.

13. Cathéter selon l'une quelconque des revendications 1 à 12, **caractérisé en ce que** le cathéter est un microcathéter avec un segment distal d'une longueur de 30 cm (11) ayant un diamètre externe maximal inférieur à 1,00 mm, de préférence de 0,75 mm au plus.

14. Cathéter selon l'une quelconque des revendications 1 à 13, **caractérisé en ce que** le cathéter est un neuro-microcathéter ayant un segment distal (11) d'une longueur d'au moins 10 cm qui a un diamètre externe maximal inférieur à 0,30 mm

15. Cathéter selon l'une quelconque des revendications 1 à 14, **caractérisé en ce que** le nombre de fils (5) varie le long du cathéter, de préférence de telle sorte que le nombre de fils (5) diminue dans la direction distale.

16. Cathéter selon la revendication 14 ou 15, **caractérisé en ce que** dans un segment proximal, la rangée de fils (5) est rendue rigide par un membre tubulaire (15) supplémentaire.

17. Cathéter selon l'une quelconque des revendications précédentes, **caractérisé en ce que** les fils (5) dans la rangée (A) à enroulement hélicoïdal sont situés à proximité les uns à côté des autres et que la rangée (A) de fils a été formée dans une course hélicoïdale dans un même mouvement, éventuellement avec un interstice (B) entre les spires de la rangée de fils.

18. Cathéter selon l'une quelconque des revendications précédentes, **caractérisé en ce que** la partie formant le corps est constituée d'une simple rangée à enroulement hélicoïdal de fils (A).

19. Cathéter selon l'une quelconque des revendications précédentes, **caractérisé en ce que** les fils (5) s'étendent de façon continue à partir de l'extrémité distale (2) jusqu'à l'extrémité proximale (4) du cathéter.
